# EUROPEAN PATENT SPECIFICATION

(11) **EP 0 608 223 B1**
(45) Date of publication and mention of the grant of the patent: **08.03.2000**
(21) Application number: 92901810.9
(22) Date of filing: 24.12.1991
(51) Int. Cl.: A61F 13/00

(54) **BANDAGE**
BANDAGE
BANDAGE

(30) Priority: 24.12.1990 GB 9028075
(43) Date of publication of application: 03.08.1994
(73) Proprietor: GARDNER, Arthur Michael Newsam, Ipplepen, Newton Abbott, Devon TQ12 5RT (GB)
(72) Inventor: GARDNER, Arthur Michael Newsam, Ipplepen, Newton Abbott, Devon TQ12 5RT (GB)
(74) Representative: Allsop, John Rowland
(86) International application number: GB9102321
(87) International publication number: WO9210983

(56) References cited:
- FR-A- 2 166 764
- FR-A- 2 533 434
- US-A- 2 096 564
- US-A- 4 926 846
- US-A- 4 926 848
- US-A- 4 926 848

## Description

This invention relates to bandages. In particular, it relates to a bandage which provides flexibility of use and extended scope of application over bandages presently available.

US-A-2 096 564 discloses a bandage comprising a flexible strip of tape having an adhesive undersurface and provided with a plurality of spaced rows of elongated apertures.

Existing bandages are typically in the form of a strip which is stored in a roll, or in the form of a tube. The tube may be shaped to conform to the particular location at which it is to be used, for example at the ankle joint. A tube, or sleeve, is generally a more convenient form than a strip since it is quick and relatively easy to apply, although its shape and size limit the scope of application.

Depending on the circumstances of use, a sleeve, in particular, may suffer the following disadvantages:-
i) it nay be unduly restrictive to movement in a certain direction in which free movement is preferable;
ii) it may not provide sufficient support, allowing too much movement in a certain direction or directions in which restricted movement is preferable;
iii) it may be uncomfortable and, in particular, may not fit adequately;
iv) it may tend to kink when the joint is flexed; or
v) it may cover an area which needs to remain open or accessible, such as a wound or bed sore.

According to the invention, there is provided a bandage comprising a flexibly stretchable first member formed of an elastic layer covered on at least one surface by a woven material which is provided with lines or areas of weakness, and a second member formed of a base material less elastic than the first member or inelastic, the second member having upstanding hooked elements for engaging the woven material of the first member; whereby to provide for controlled flexibility over predetermined areas of the first member upon engagement therewith.

The first member may be easily extensible and the lines or areas of weakness may be local in extent. Such lines or areas of weakness provide means for controlling the stretching of the first member in diverse local areas and in specific directions according to the requirements of a particular application. Lines or areas of weakness are preferably slits or cuts only partially through the first member, but may be made through the entire thickness of the first member. The lines or areas of weakness may be introduced in situ and/or during manufacture. Lines of weakness are preferably generally parallel to the longitudinal axis of the limb to be bandaged. The bandage may thus be adapted to allow easy movement in specific areas and in specific directions since the first member will stretch in a direction generally normal to each slit. The use of a series of lines of weakness in the form of a lattice allows stretching in all directions.

In a preferred embodiment, the areas of weakness may be apertures which allow access to a wound site, for example, or which prevent wrinkling of the bandage during flexing of a joint.

The second member provides means for fastening the bandage when the hooked elements engage woven material of the first bandage.

One or more pieces of the second member may be used to strengthen the bandage by limiting or preventing stretching of the first member. One or more pieces of the second member by also be used to seal unwanted lines or areas of weakness in the first member. The elastic layer is preferably of Neoprene or similar material. The woven material is preferably attached to the elastic layer by adhesive, although alternative methods, such as heat sealing for example, may be used.

Still more preferably, means for holding inserts in position may be provided. The means may be pockets in the woven material which can hold inserts such as protective pads and plates, or inflatable pads for use with impulse-compression pads for local treatment of joints or tissues or activation of physiological venous pumps, eg. of the hand, foot, calf, knee or thigh.

Alternatively, inserts may be located by positioning the inserts next to the first member and fixing in place by one or more pieces of the second member. In this manner inserts may be located on either side of the first member as required.

The bandage is suitably manufactured in the form of a sheet, but preferably it is in the form of a tubular bandage of a particular joint shape. Strips of bandage may be obtained by cutting a sheet into the required length and/or width. Tubular bandages may also be made in situ by sealing the ends of a sheet of the first member with one or more strips of the second member. A sheet may thus be closed by the one or more strips of the second member so as to form a cylinder.

The bandage may have an adhesive layer on part or all of at least one surface of the first member. Such an adhesive layer would improve protection of damaged ligaments or structural tissue by adhering to the skin surface.

According to a further aspect of the invention, there is provided a method of manufacturing a bandage comprising: manufacturing a flexibly stretchable first member by providing a layer of elastic material, with a layer of woven material attached to at least one surface of said layer and cutting slits through part or all of the first member thus formed; and controlling the flexibility of the first member by fastening one or more pieces of a second member formed of a base material which is inelastic or less elastic than the first member to a predetermined area or areas of the first member by engaging hooked elements upstanding from the second member with the woven material of the first member.

Applications of the bandage the subject of the invention include:
i) circumstances where support is required, such as in the protection of damaged ligaments, where appropriately position inhibition means can be used;
ii) to hold impulse-compression pads inside a tubular bandage for local treatment of joints and tissues;
iii) where rapid and unskilled placement of dressings with localised pressure is required, such as for preventing the bleeding of a wound in an emergency;
iv) where ventilation of pressure sores or relief of pressure is required; and
v) in the constructions of an immobilisation cast for body or limb, with the addition of a quick-setting material such as an alginate or plaster of Paris.

The thickness of the bandage is not critical to the invention since it should be suited to the particular material used and the specific application envisaged. A typical useful thickness is from 1.5 mm to 8 mm.

The numbers, sizes, shapes, locations, orientations and degrees of the areas of elastic inhibition and assistance govern the shape and characteristics of the bandage so that the bandage can be tailor-made for a specific application. Thus a bandage can be manufactured for application, not only for a particular site, but also for a particular purpose, and applied with speed and ease even by an unskilled person. Likewise, a bandage can be altered or adjusted before or during fitting to suit a special requirement. The bandage should conform to the particular application in that it must not compromise circulation.

The bandage and/or inhibition means may be colour-coded to indicate the degree to which they are elastic.

The invention will now be described by way of example with reference to the accompanying drawings, in which:
Fig 1 is a schematic transverse section of a bandage according to the invention;
Fig 2 is a schematic plan view of another bandage according to the invention;
Fig 3 is a schematic transverse section of the bandage of Fig 2 viewed in direction A-A;
Fig 4 is a schematic plan view of a further bandage according to the invention;
Fig 5 is a schematic plan view of a stretched section of the bandage of Fig 4; and
Fig 6 is a schematic side view of a yet further bandage according to the invention.

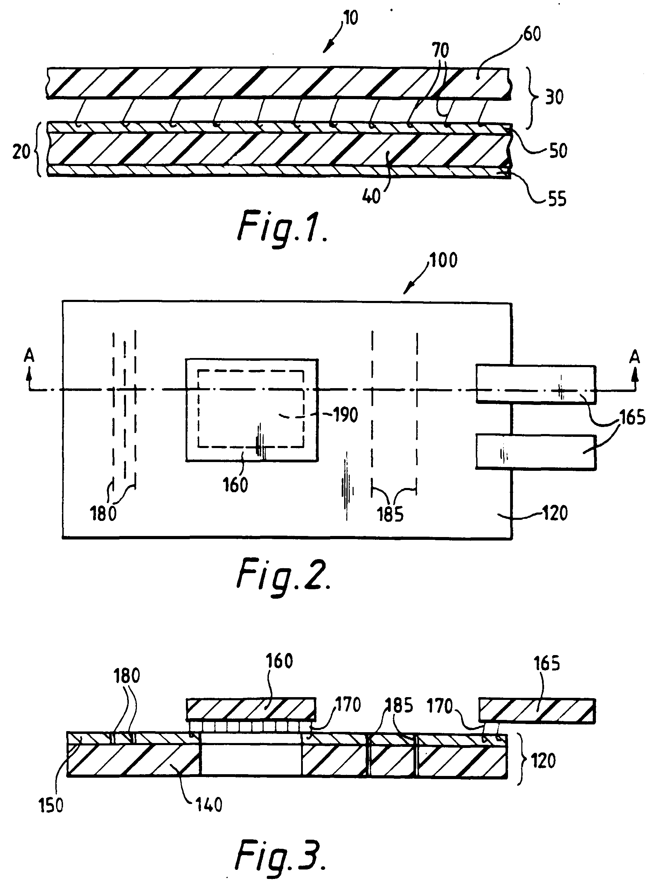

In Fig 1, there is shown a bandage 10 comprising a first member 20 and a second member 30. The first member 20 has a layer 40 with a covering layer on each side, the first member 20 being provided with areas or lines of weakness preferably in the form of slits as will be described more clearly in later embodiments of the invention. The covering layer on one side provides a comfort layer 55 for contact with the body and may also be used for location of inflation pads by a further second member. The covering layer 50 on the other side is engaged by hooked elements 70 extending from layer 60 of the second member 30. Layer 40 is an elastic material such as Neoprene with covering layers 50 and 55 being woven material. In use the woven material 50, 55 is attached to the elastic material 40 during manufacture, by adhesive for example, so that the first member 20 can be stretched around the part of the body to be bandaged. Layer 60 is less elastic than the first member or may be inelastic, being a moulded plastics material for example. In use, the hooked, elements 70 engage the woven material 50, preventing stretching of first member 20 at the points of attachment.

Figs 2 and 3 show an embodiment in which a bandage 100 has slits 180, 185 cut in its first member 120, and an aperture 190. Slits 180 are partial thickness, being cut through the woven material 150 only. Slits 185 are full thickness, being cut through both layers of the first member 120, comprising woven material 150 and elastic material 140 only in this embodiment.

Strips of inelastic material 165 are provided for securing the bandage and pieces of inelastic material 160 is placed around the aperture 190 so as to provide a local inelastic region which prevents unwanted deformation of the bandage.

Second member pieces 160, 165 may be Velcro (registered trade mark) or other similar inelastic material having hooked elements. The first member is, for example, wetsuit material, which may be 3 mm Neoprene lined with a 0.5 mm thick woven material. It will be appreciated that other materials could be used. In particular woven material 150 could be a piled fabric, such as plush, or could have looped elements which engage with the hooked elements 170.

In use, sheets of bandage 100 are placed around the part of the body to be bandaged and are secured in place by strips of inelastic material 165. Slits 180 in the sheet provide lines of weakening, so that the sheet stretches generally normal to these lines. Instead of being manufactured as a sheet, the bandage may be manufactured in the form of a tube. In this case, slits are provided around arcs of the circumference of inelastic material. Unwanted slits may be sealed by strips of pieces of inelastic material. Further strips of inelastic material may be positioned where extra support or strengthening is required, such as along damaged ligaments or across incompletely healed fractures.

This example uses inelastic material for securing or strengthening, but it is possible to use material which is less elastic than that of the first member.

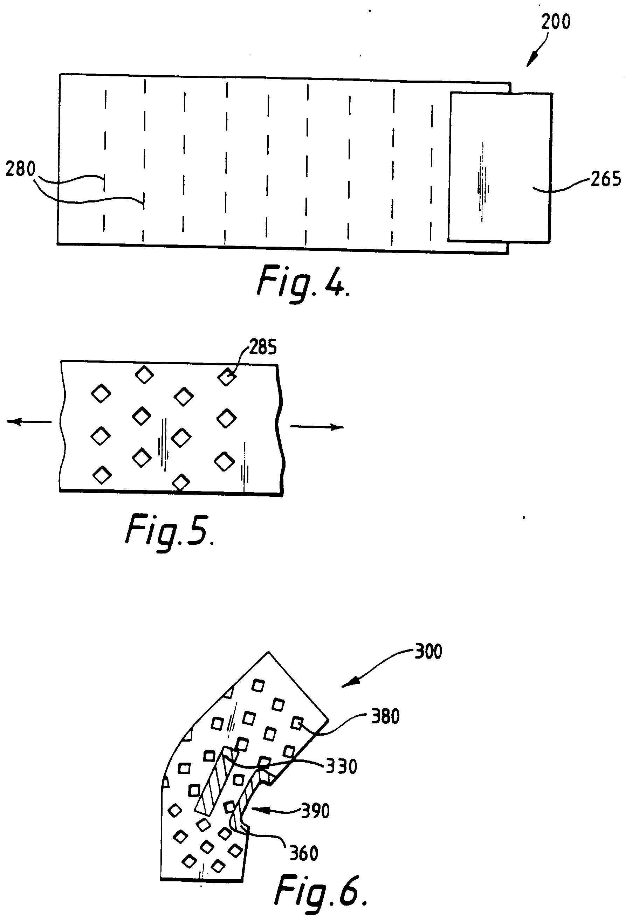

In another embodiment of the invention, the slits in the first member 120 of the bandage of Fig 3 are in the form of multiple slits 280 arranged in a "bricked" layout in the bandage 200 illustrated in Fig 4. These slits 280 deform when stretched around a limb during application as shown in Fig 5. The multiple slits become a lattice of diamond shapes 285 when stretched as indicated by the arrows. The use of this form enables stretching in all directions. Improved stretching is thereby achieved over the whole of the lattice region.

The bandage 200 may be closed by a longitudinal strip of the second member 265 to form a tubular bandage.

The slits are generally closely spaced along the longitudinal axis of the limb to be supported. This weakens the force exerted by the first member so that by itself it exerts only minor constricting force or the limb.

Fig 6 shows an embodiment in which the bandage according to the invention has been manufactured in the form of a tubular support bandage 30 and shaped to conform to a knee joint. Slits 380 have been cut in the first member of the bandage in the region where stretching is required and have deformed to a diamond lattice. Further support to ligaments, for instance, is provided in the form of inelastic or less elastic regions obtained by the addition of strips of the second member 330. If necessary, an aperture 390 can prevent wrinkling of the bandage, the aperture being maintained in shape by a piece of the second member 360. The strips 330 may also be used to close a slit 380 if it is not required.

A versatile bandage and method of manufacturing a bandage have thus been provided which provide quick and easy application without the requirement of a large range of custom-fit bandages.

## Claims

1. A bandage comprising a flexibly stretchable first member (20,120) formed of an elastic layer covered on at least one surface by a woven material (50,15), wherein the first member (20,120) has lines or areas of weakness (180,185), and a second member (30,160) formed of a base material less elastic than the first member (20,120) or inelastic, the second member (30,160) having upstanding hooked elements (70,170) for engaging the woven material of the first member (20,120); whereby to provide for controlled flexibility over predetermined areas of the first member (20,120) upon engagement therewith.

2. A bandage according to Claim 1 in which the lines or areas of weakness (180,185) are slits through part or all of the first member.

3. A bandage according to Claim 2 in which the slits (180,280,380) are arranged in a lattice form of staggered rows.

4. A bandage according to any one of Claims 1 to 3 in which the second member (30,160) provides means for strengthening the bandage.

5. A bandage according to any one of Claims 1 to 4 in which the elastic layer (20,120) is Neoprene or similar material.

6. A bandage according to any one of Claims 1 to 5 in which the first member is in the form of a strip, sheet or tube.

7. A bandage according to any one of Claims 1 to 7 in which the first member (20,120) further comprises an adhesive layer on part or all of at least one surface.

8. A method of manufacturing a bandage comprising:
manufacturing a flexibly stretchable first member (20,120) by providing a layer of elastic material, with a layer of woven material (50,150) attached to at least one surface of said layer and cutting slits (180,280,380) through part or all of the first member thus formed; and
controlling the flexibility of the first member (20,120) by fastening one or more pieces of a second member (30,160) formed of a base material which is inelastic or less elastic than the first member (20,120) to a predetermined area or areas of the first member by engaging hooked elements (70,170) upstanding from the second member (30,160) with the woven material (50,150) of the first member (20,120).

## Patentansprüche

1. Bandage bestehend aus einem flexibel dehnbaren ersten Element (20, 120), welches von einer elastischen Lage gebildet ist, die an wenigstens einer Oberflächenseite durch ein Gewebe (50, 15) abgedeckt ist, wobei das erste Element (20, 120) Schwächungslinien oder -bereiche (180, 185) aufweist, und aus einem zweiten Element (30, 160), welches aus einem Grundmaterial mit einer im Vergleich zum ersten Element (20, 120) geringeren Elastizität oder aus einem nichtelastischen Material hergestellt ist, wobei das zweite Element (30, 160) nach oben wegstehende hakenförmige Elemente (70, 170) für den Eingriff mit dem Gewebematerial des ersten Elementes (20, 120) besitzt, um durch Eingriff eine kontrollierte Flexibilität über vorbestimmte Bereiche des ersten Elementes (20, 120) zu erreichen.

2. Bandage nach Anspruch 1, dadurch gekennzeichnet, daß die Schwächungslinien oder-bereiche (180, 185) Schlitze sind, die teilweise oder vollständig durch das erste Element hindurchgehen.

3. Bandage nach Anspruch 2, dadurch gekennzeichnet, daß die Schlitze (180, 280, 380) gitterartig in versetzten oder aufeinander folgenden Reihen vorgesehen sind.

4. Bandage nach einen der Ansprüche 1 - 3, dadurch gekennzeichnet, daß das zweite Element (30, 160) Mittel für eine Verstärkung der Bandage zur Verfügung stellt.

5. Bandage nach einem der Ansprüche 1 - 4, dadurch gekennzeichnet, daß die elastische Schicht (20, 120) Neopren oder ein ähnliches Material ist.

6. Bandage nach einem der Ansprüche 1 - 5, dadurch gekennzeichnet, daß das erste Element die Form eines Streifens, eines Flachmaterials oder eines Schlauches aufweist.

7. Bandage nach einem der Ansprüche 1 - 7, dadurch gekennzeichnet, daß das erste Element (20, 120) auf wenigstens einer Oberflächenseite teilweise oder vollständig mit einer Klebeschicht versehen ist.

8. Verfahren zum Herstellen einer Bandage, wobei das Verfahren umfaßt: das Herstellen eines flexibel dehnbaren ersten Elementes (20, 120) durch Bereitstellen einer Schicht eines elastischen Materials, wobei eine Lage aus einem Gewebematerial (50, 150) an wenigstens einer Oberflächenseite der erwähnten Schicht befestigt wird und Schlitze (180, 280, 380) teilweise durchgehend oder vollständig durchgehend durch das so hergestellte erste Element eingebracht werden; und
das Steuern der Flexibilität des ersten Elementes (20, 120), durch Befestigen eines oder mehrerer Stücke eines zweiten Elementes (30, 160), welches aus einem Grundmaterial gefertigt ist, das nicht elastisch oder eine Elastizität kleiner als die Elastizität des ersten Elementes (20, 120) aufweist, und zwar an einem vorbestimmten Bereich oder an vorbestimmten Bereichen des ersten Elementes durch Verbindung von hakenförmigen Elementen (70, 170), die von dem zweiten Element (30, 160) wegstehen, mit dem Gewebematerial (50, 150) des ersten Elementes (20, 120).

## Revendications

1. Bandage comprenant un premier élément souple étirable (20, 120) formé d'une couche élastique recouverte sur au moins une surface par un materiau tisse (50, 15), ledit premier élément (20, 120) comportant des lignes ou des zones de moindre résistance (180, 185), et un second élément (30, 160) formé d'un matériau de base moins élastique que le premier élément (20, 120), ou inélastique, et muni de crochets (70, 170) destinés â s'accrocher au matériau tissé du premier élément (20, 120), afin de'obtenir une souplesse ajustable dans des zones prédéterminées du premier élément (20, 120) lors de l'accrochage â celui-ci.

2. Bandage selon la revendication 1, dans lequel les lignes ou zones de moindre résistance (180, 185) sont des fontes â travers tout ou partie due premier élément.

3. Bandage selon la revendication 2, dans lequel les fentes (180, 280, 380) sont disposées sous forme de grilles de rangées décalées.

4. Bandage selon l'une quelconque des revendications 1 â 3, dans lequel le second élément (30, 160) est muni de moyens permettant de consolider le bandage.

5. Bandage selon l'une quelconque des revendications 1 â 4, dans luquel la couche élastique (20, 120) est due Néoprène ou un matériou similaire.

6. Bandage selon l'une quelconque des revendications 1 â 5, dans lequel le premier élément a la forme d'une bande, d'une feuille ou d'un tube.

7. Bandage selon l'une quelconque des revendications 1 â 6, dans lequel le premier élément (20, 120) comprend en outre une couche adjésive sur tout ou partie d'au moins une surface.

8. Procédé de fabrication d'un bandage consistant â : fabriquer un premier élément souple étirable (20, 120) en adjoignant â une couche de matériau élastique une couche de matériau tissé (50, 150) fixée â au moins une surface de ladite couche et en coupant des fentes (180, 280, 380) â travers tout ou partie du premier élément ainsi formé ; et a ajuster la souplesse du premier élément (20, 120) en fixant un ou plusieurs morceaux d'un second élément (30, 160) fait d'un matériau de base inélastique ou moins élastique que le premier élément (20, 120) sur une ou des zones prédéterminées du premier élément, en agrippant des crochets (70, 170) places sur le second élément (30,160) au matériau tissé (50, 150) du premier élément (20, 120).
